# EUROPÄISCHE PATENTSCHRIFT

(11) **EP 2 595 583 B1**
(45) Veröffentlichungstag und Bekanntmachung des Hinweises auf die Patenterteilung: **20.07.2016**
(21) Anmeldenummer: 12714616.5
(22) Anmeldetag: 30.03.2012
(51) Int. Cl.: A61F 5/058

(54) **TEMPORÄRE SCHIENE**
TEMPORARY SPLINT
ATTELLE TEMPORAIRE

(30) Priorität: 04.08.2011 DE 102011109431
(43) Veröffentlichungstag der Anmeldung: 29.05.2013
(73) Patentinhaber: BSN medical GmbH, 20253 Hamburg (DE)
(72) Erfinder: SCHÜTZ, Patrick, 21075 Hamburg (DE); JORISSEN, Koen, 20253 Hamburg (DE)
(74) Vertreter: Uexküll & Stolberg
(86) Internationale Anmeldenummer: PCT/EP2012/055847
(87) Internationale Veröffentlichungsnummer: WO 2013/017299

(56) Entgegenhaltungen:
- EP-A1- 0 359 635
- EP-A1- 2 065 019
- FR-A1- 2 671 484
- FR-A1- 2 718 346
- US-A- 2 486 687

## Beschreibung

Die vorliegende Erfindung betrifft eine Schiene zum Stützen eines Fußes und Schienbeins gemäß dem Oberbegriff des Anspruchs 1.

Eine derartige Schiene ist beispielsweise aus der EP 2 065 019 A1 bekannt, wobei die Schiene gemäß einem ersten dort beschriebenen Ausführungsbeispiel aus einem Flächenkörper besteht, der derart gebogen ist, dass die Schiene ein erstes Körperelement und ein zweites Körperelement mit jeweils U-förmigem Querschnitt aufweist, wobei die Längsachsen der Körperelemente senkrecht zueinander verlaufen, also einen Schienenwinkel von 90° einschließen. Dadurch kann beispielsweise ein Arm in der Schiene aufgenommen werden, wobei sich der Ellenbogen durch eine zwischen den Körperelementen vorgesehene Öffnung heraus erstrecken kann.

Außerdem ist in der EP 2 065 019 A1 ein weiteres Ausführungsbeispiel offenbart, bei dem ein Körperelement einen ersten Anlageabschnitt und einen zweiten Anlageabschnitt aufweist, die in einem Winkel zueinander angeordnet sind, wobei das Material des Körperelements wiederum ein flächenförmiges biegsames flexibles Material ist und der Winkel zwischen den Anlageabschnitten durch einen zusätzlichen, ebenfalls aus einem flexiblen Material geformten Stützkörper festgelegt wird.

Die in der EP 2 065 019 A1 beschriebenen Schienen sind zunächst mit dem Vorteil verbunden, dass die jeweilige Schiene nicht im fertig geformten Zustand transportiert werden muss, sondern als flächenförmiger Zuschnitt, der erst unmittelbar vor der Benutzung aufgefaltet wird. Damit beansprucht die Schiene im nicht-aufgefalteten Zustand relativ wenig Platz und kann so auch in Rettungswagen oder dergleichen transportiert bzw. mitgeführt werden.

Die US 2,486,687, von der die vorliegende Erfindung ausgeht, beschreibt eine Schiene aus Wellpappe zur Ruhigstellung eines Arms oder Beins eines Patienten. Dabei reicht das Körperelement, das zur Aufnahme des Schienbeins des Patienten vorgesehen ist, bis unterhalb des Kniegelenks des Patienten. Ferner ist ein zusätzliches, optionales Verlängerungselement zur Verlängerung des Beinabschnitts der Schiene über das Kniegelenk hinaus vorgesehen, das den Beinabschnitt umgreift und aufnimmt.

Allerdings ist es erforderlich, eine derartige Schiene mit Hilfe eines Verbandmaterials am entsprechenden Körperabschnitt des Patienten zu befestigen. Dabei ergibt sich zum einen das Problem, dass zuverlässig verhindert werden muss, dass sich die Schiene während des Tragens von dem Körperabschnitt löst oder sich das Verbandmaterial gegenüber der Schiene verschiebt.

Außerdem ergibt sich bei einer mit Hilfe von Verbandmaterial befestigten derartigen Schiene das Problem, dass der Bereich zwischen der Schiene und dem Körperabschnitt des Patienten nicht hinreichend hinterlüftet ist und aus einer Wunde austretendes Wundexsudat nicht ohne Weiteres abgeführt werden kann. Dies kann dazu führen, dass sich beim Patienten Mazerationen bilden.

Daneben besteht die Gefahr, dass dann, wenn das flexible Material der Körperelemente Pappe oder dergleichen ist, dieses infolge von Schweiß und Wundexsudat aufweicht.

Darüber hinaus ergibt sich bei der bekannten Schiene das Problem, dass in dem Bereich des gestützten Körperabschnitts, der an der Schiene anliegt, insbesondere dann, wenn das Material der Schiene bereits Feuchtigkeit aufgenommen hat, Entzündungen entstehen können.

Ein weiteres Problem ergibt sich bei der bekannten, zwei Körperelemente aufweisenden Schiene dadurch, dass dann, wenn die Schiene aus einem einstückig ausgebildeten Zuschnitt geformt ist, eine Überlapp- Verbindung hergestellt wird, um freie Enden des Zuschnitts miteinander zu verbinden. An dieser Stelle ergibt sich aber die Gefahr, dass es im Bereich der ÜberlappVerbindung am gestützten Körperabschnitt zu Druckstellen kommen kann.

Ein weiterer Nachteil bei der bekannten Schiene ist, dass sie nicht hinreichend stabil ist, um den Bereich des Schienbeins und des Fußes eines Patienten zu stützen, ohne beim Auftreten des Patienten auf den Boden Schaden zu nehmen. Hier hat sich gezeigt, dass die Schiene nicht ausreichend ist. Außerdem werden Stöße durch das Auftreten ungedämpft auf den Fuß und das Schienbein übertragen, was ebenfalls unerwünscht ist.

Wenn das Körperelement der Schiene zwei in einem Winkel zueinander angeordnete Anlageabschnitte aufweist, ist es erforderlich, einen Stützkörper vorzusehen, der den Winkel zwischen den Anlageabschnitten festlegt. Wenn dieser Stützkörper ebenfalls aus einem flexiblen flächenförmigen Material hergestellt ist, kann es dazu kommen, dass dieser nicht die erforderliche Steifigkeit aufweist oder das Volumen des Stützkörpers unangemessen groß werden muss.

Schließlich muss das flächenförmige Material für die Schiene in eine Vorzugsrichtung biegbar sein, während in einer Richtung senkrecht dazu eine hohe Biegesteifigkeit wünschenswert ist. In diesem Zusammenhang hat sich gezeigt, dass herkömmliches Pappmaterial diesen Anforderungen häufig nicht genügt. Es besteht daher ein Bedarf, ein verbessertes Material für die Schienen bereitzustellen.

Insgesamt ist es die Aufgabe der vorliegenden Erfindung, eine Schiene der eingangs genannten Art bereitzustellen, die nicht die zuvor genannten Nachteile aufweist.

Erfindungsgemäß wird die eingangs genannte Aufgabe gelöst durch eine Schiene gemäss Anspruch 1.

Durch das Zusatzelement wird die Stabilität der Schiene im Bereich dieses Körperelements erhöht. Dies ist insbesondere vorteilhaft, wenn eines der Körperelemente an der Fußsohle anliegt und der Patient trotz der Schiene mit dieser auftreten soll. Dann ist in dem Bereich dieses Körperelements eine erhöhte Stabilität erforderlich, die durch das Zusatzelement gewährleistet wird.

Gemäß einem weiteren Aspekt der vorliegenden Erfindung weist die Schiene zum Stützen eines Körperabschnitts eines Patienten ein Körperelement aus einem flächenförmigen flexiblen Material auf, das eine zum Körperabschnitt weisende Anlageoberfläche und eine von der Anlageoberfläche wegweisende Außenoberfläche aufweist, wobei die Anlageoberfläche eine antimikrobiell wirkende Beschichtung aufweist.

Durch die antimikrobiell wirkende Beschichtung wird die Gefahr von Entzündungen am von der Schiene gestützten Körperabschnitt des Patienten signifikant vermindert.

In diesem Zusammenhang ist unter dem Begriff "flächenförmig" im Sinne der vorliegenden Erfindung ein Körper zu verstehen, dessen größte Abmessung in einer Ebene die Abmessung in der Richtung senkrecht zu dieser Ebene um wenigstens eine Größenordnung, vorzugsweise jedoch um mehr als eine Größenordnung übersteigt.

In einer bevorzugten Ausführungsform kann die antimikrobiell wirkende Beschichtung als Acetat-Träger ausgebildet sein, wobei dieser in weiter bevorzugter Weise auf die Anlageoberfläche aufkaschiert ist. Eine derartige Beschichtung wirkt außerordentlich effizient gegen Krankheitserreger.

Alternativ ist es auch möglich, dass die antimikrobiell wirkende Beschichtung als Wirksubstanz Dialkylcarbamoylchlorid (DACC) und/oder Polyhexamethylenbiguanid (PHMB) und/oder Silber aufweist. Dabei kann die Wirksubstanz in ein Vlies, Gewebe, Gestrick oder Gewirk eingebracht sein, das auf der Anlageoberfläche aufkaschiert ist.

Diese Wirksubstanzen sind ebenfalls sehr geeignet, Krankheitserreger, die zu Entzündungen im Bereich des gestützten Körperabschnitts führen können, zu bekämpfen. Durch das Einbringen der Wirksubstanz in ein Vlies, Gewebe, Gestrick oder Gewirk ist diese dauerhaft auf der Anlageoberfläche vorhanden, sodass die antimikrobielle Wirkung über einen langen Zeitraum besteht. Dieser Effekt ist insbesondere dann gegeben, wenn das Vlies, Gewebe, Gestrick oder Gewirk bereits bei dessen Herstellung mit der Wirksubstanz ausgerüstet wird.

Gemäß einem weiteren Aspekt der vorliegenden Erfindung weist die Schiene zum Stützen eines Körperabschnitts eines Patienten ein Körperelement aus einem flächenförmigen flexiblen Material auf, das eine zum Körperabschnitt weisende Anlageoberfläche und eine von der Anlageoberfläche wegweisende Außenoberfläche aufweist, wobei auf der Anlageoberfläche eine Absorptionsschicht zur Aufnahme von Flüssigkeit und Wundexsudat aufgebracht ist.

Durch die Absorptionsschicht wird erreicht, dass Wundexsudat, das von einer Wunde im Bereich des gestützten Körperabschnitts abgesondert wird, nicht in das flexible Material der Schiene eindringen kann und dieses gegebenenfalls aufweicht, sondern von der Absorptionsschicht gespeichert wird. Dadurch werden zwei Probleme vermieden. Zum einen wird Wundexsudat abgeführt, sodass die Gefahr von Mazerationen beim Patienten vermindert wird, und zum anderen wird dennoch gewährleistet, dass die temporäre Schiene durch abgegebenes Wundexsudat nicht an Stabilität verlieren kann.

Vorzugsweise ist die Absorptionsschicht als Schaum ausgeführt, dessen Hohlräume zur Aufnahme des Exsudats dienen. Insbesondere kann es sich dabei um einen offenzelligen Polyurethanschaum handeln, dessen Dichte vorzugsweise zwischen 30 und 110 kg/m³ liegt und/oder dessen Dicke zwischen 2,5 und 15 mm liegt. Ein solcher Schaum hat für normale Anwendungen eine hinreichend hohe Speicherkapazität und trägt dennoch nicht zu stark auf.

Alternativ zu Schaum kann die Absorptionsschicht auch als Vliesverbundschicht ausgebildet sein, wobei die VliesVerbundschicht eine Viskoseschicht und eine auf der von dem Körperelement abgewandten Oberfläche der Viskoseschicht vorgesehene Polyesterschicht aufweist.

Bei einer derartig aufgebauten Absorptionsschicht wird sichergestellt, dass das einmal darin aufgenommenes Exsudat nicht ohne weiteres wieder abgegeben wird, was durch die auf der zum Körperabschnitt weisenden Seite der Verbundschicht vorgesehene Polyesterschicht gewährleistet wird.

Gemäß einem anderen Aspekt der vorliegenden Erfindung weist die Schiene zum Stützen eines Körperabschnitts eines Patienten ein Körperelement aus einem flächenförmigen flexiblen Material auf, das eine zum Körperabschnitt weisende Anlageoberfläche und eine von der Anlageoberfläche wegweisende Außenoberfläche aufweist, wobei wenigstens auf einem Teil der Außenoberfläche eine Schicht aufgebracht ist, die mit einem Gewebe, insbesondere Verbandmaterial, eine gegenüber dem Material des Körperelements erhöhte Haftreibung hat.

Durch den hohen Haftreibungskoeffizienten der auf der Außenoberfläche aufgebrachten Schicht gegenüber einem Verbandmaterial wird sichergestellt, dass dieses sich nicht mehr verschieben kann, wenn die Schiene am Körperabschnitt eines Patienten mit Hilfe eines Verbandmaterials befestigt worden ist. Dadurch wird eine dauerhafte Fixierung sichergestellt. Alternativ kann die Schicht als Lackierung oder als aufkaschierte Schicht ausgebildet sein.

Insbesondere ist es vorteilhaft, wenn die Schicht als Folie oder als gitterförmig verlaufendes Material, das in bevorzugter Weise aus einem polymeren Werkstoff geformt ist, ausgebildet ist. Bei einer solchen Ausführung ergibt sich ein guter Eingriff zwischen Schicht einerseits und Verbandmaterial andererseits, so dass sich Letzteres nicht gegenüber der Schiene verschieben kann, wenn es unter Zugspannung steht.

In einer alternativen Ausführungsform ist es auch möglich, das Haken-Element einer Haken-Schlaufen-Verbindung bzw. eines "Klettverschlusses" auf der Außenoberfläche aufzubringen, das ebenfalls in zuverlässiger Weise ein Verbandmaterial am Körperelement fixieren kann.

Durch Verbindungselemente, die die beiden Körperelemente miteinander verbinden, wird ermöglicht, dass die zueinander weisenden Kanten der Körperelemente einen Stumpfstoß bilden, die Körperelemente also in einer gemeinsamen Ebene verlaufen, ohne dass eine Überlappverbindung auftritt, die im Bereich des Körperabschnitts des Patienten, der von der Schiene gestützt wird, zu Druckstellen führen könnte.

Sich von den Außenkanten des Körperelements wegerstreckenden Laschen können auf der von der Längsachse abgewandten Seite des Körperabschnitts miteinander verbunden werden, so dass der Körperabschnitt vollständig von dem Körperelement umschlossen ist und die Schiene zuverlässig am Körperabschnitt fixiert wird. Dies ist insbesondere dann von Vorteil, wenn die Schiene dazu genutzt werden soll, den Ober- und Unterarm eines Patienten zu stützen. Dann können die Laschen an dem Körperelement vorgesehen sein, in das der Oberarm eingesetzt ist, so dass die Laschen dann den Oberarm innerhalb des entsprechenden Körperelements wirksam festlegen.

Weiterhin ist es vorteilhaft, wenn die Laschen senkrecht zur Längsachse des Körperelements verlaufen, da dann Reaktionskraft, die sich beim Befestigen der freien Enden der Laschen miteinander ergibt, in Erstreckungsrichtung der Laschen verläuft, so dass in diesen keine Scherkräfte auftreten.

Des weiteren ist es vorteilhaft, wenn die Laschen Falzlinien aufweisen, die sich parallel zur Längsachse des Körperelementes erstrecken, da die Lasche dann in einfacher Weise um den zu stützenden Körperabschnitt gebogen werden kann.

Aufgrund der Fixierung durch die Laschen ist es nicht erforderlich, dass das Körperelement den zu stützenden Körperabschnitt in großem Maße umgibt. Deswegen können die Außenkanten des Körperelements geneigt zur Längsachse verlaufen.

Wenn der Winkel, den die Längsachsen der Körperelemente miteinander einschließen, also der Schienenwinkel, zwischen 80° und 100° liegt und vorzugsweise bei 90°, kann die Schiene besonders gut zur Stützung eines Arms oder des Fußgelenks eingesetzt werden.

Ferner ist es vorteilhaft, wenn im Bereich der von den Längsachsen entfernt liegenden Kanten der Körperelemente Aussparungen, die vorzugsweise eine längliche Form haben, wobei die Richtung der größten Erstreckung parallel zur Längsachse des Körperelements verläuft, vorgesehen sind. Dies ermöglicht, die Schiene mit Hilfe von Gurten am zu stützenden Körperabschnitt festzulegen, wobei sich die Gurte dann durch die Aussparungen erstrecken.

Schließlich hat es sich als vorteilhaft erwiesen, wenn im Bereich der Verbindung der Körperelemente entweder sternförmig angeordnete Einschnitte oder Schaumstoffelemente vorgesehen sind. Dadurch wird verhindert, dass in dem Bereich der Schiene, an der sie an einem Gelenk anliegt, Druckstellen entstehen.

Durch einen stabförmigen Stützkörper, der den Winkel zwischen den Längsachsen der Körperelemente bzw. der Anlageabschnitte festlegt, wird vermieden, dass ein kompliziert aufgebauter, aus flexiblem Material gefalteter Stützkörper verwendet werden muss, der möglicherweise keine ausreichende Stabilität gewährleistet oder ein großes Volumen einnimmt.

Vorzugsweise ist der Stützkörper aus einem thermoplastischen Kunststoff gebildet, dessen Erweichungstemperatur im Bereich zwischen 50°C und 200°C liegt, vorzugsweise zwischen 60°C und 170°C und am meisten bevorzugt zwischen 70°C und 140°C. Dann kann der Winkel nach einem einfachen Erwärmen des Stützkörpers durch Biegen vorgegeben werden, und anschließend lässt man den Stützkörper erkalten, so dass dieser Winkel fixiert ist. Dadurch kann ein Arzt oder Sanitäter in einfacher Weise den gewünschten Winkel einstellen, ohne dass hierzu herstellerseitig besondere Maßnahmen erforderlich sind.

In einer bevorzugten Ausführungsform ist der Stützkörper aus einem Vliesstoff gebildet, der in weiter bevorzugter Weise aus Baumwolle, Polypropylen und Polyester gebildet ist. Dabei ist es besonders bevorzugt, wenn der Vliesstoff 40% Baumwolle, 30% Polypropylen und 30% Polyester aufweist. Diese Angaben beziehen sich dabei auf die Prozentanteile des Gewichts des Vliesstoffes.

Wenn die Grammatur des Vliesstoffes, aus dem der Stützkörper geformt ist, größer als 500 g/m² ist, weist dieser die notwendige Festigkeit auf.

Des Weiteren ist es vorteilhaft, wenn der Stützkörper ein Verbindungselement aufweist, das mit dem ersten Abschnitt und dem zweiten Abschnitt beanstandet zu der Verbindung zwischen diesen verbunden ist, um den Schienenwinkel festzulegen. Durch das zusätzliche Verbindungselement wird vermieden, dass die Abschnitte des Stützelements, die an den Anlageabschnitten anliegen, später während der Benutzung auseinander oder zusammen gebogen werden können und sich der Schienenwinkel ändert.

Außerdem ist es bevorzugt, wenn Befestigungseinrichtungen zwischen dem Stützkörper und der Außenoberfläche des bzw. der Körperelemente vorgesehen sind, so dass der Stützkörper lösbar damit verbunden ist. Dies erleichtert die Montage der Schiene.

Eine besondere Ausführungsform der Befestigungselemente ist derart ausgebildet, dass an der zur Außenoberfläche weisenden Seite des Stützkörpers Befestigungselemente vorgesehen sind, die einen ersten Abschnitt aufweisen, der mit dem Stützelement verbunden ist, sich davon weg erstreckt und eine erste Abmessung senkrecht zu seiner Erstreckungsrichtung aufweist, wobei an dem vom Stützkörper entfernten Ende des ersten Abschnitts ein zweiter Abschnitt vorgesehen ist, der eine zweite Abmessung senkrecht zu dessen Erstreckungsrichtung aufweist, wobei die zweite Abmessung größer als die erste Abmessung ist und wobei in dem Körperelement den Befestigungselementen zugeordnete Öffnungen vorgesehen sind. Dabei sind in besonders bevorzugter Weise die Befestigungselemente aus einem elastischen Kunststoff, insbesondere Silikon, ausgebildet.

Derartige pilzförmig ausgebildete Befestigungselemente können in die Öffnungen in den Körperelementen eingedrückt werden, so dass auf diese Weise das Stützelement in einfacher Weise befestigt werden kann.

Alternativ ist es auch möglich, das Stützelement über eine Haken-Schlaufen-Verbindung, also einen Klettverschluss, Körperelemente zu befestigen.

Eine Schiene mit einem mäanderförmigen Streifen aufweisenden Material hat eine hohe Stabilität in Verlaufsrichtung der Streifen, während es dennoch möglich ist, das Material senkrecht zur Verlaufsrichtung zu biegen.

Insbesondere dann, wenn das Material der Streifen flächenförmig ist und sich senkrecht zur Verlaufsrichtung der ersten und zweiten Schichten erstreckt, ist das Material einerseits sehr steif, weist aber ein geringes Gewicht auf, was den Tragekomfort erhöht. Die erste und zweite Schicht können aus Pappe oder Polypropylen gebildet sein. Es ist aber auch denkbar, dass die erste oder zweite Schicht ein Vlies aufweisen. Um die Stabilität weiter zu erhöhen, ist es bevorzugt, dass zwischen den mäanderförmig verlaufenden Streifen noch gradlinige Streifen im Bereich zwischen den Schichten angeordnet sind.

Im Folgenden wird die vorliegende Erfindung anhand Zeichnung erläutert, die lediglich bevorzugte Ausführungsbeispiele zeigt, wobei
- Fig. 1: eine erste perspektivische Ansicht einer ersten Schiene zeigt,
- Fig. 2: eine zweite perspektivische Ansicht des ersten Ausführungsbeispiels zeigt,
- Fig. 3: einen Zuschnitt für die in den Fig. 1 und 2 gezeigte Ausführungsbeispiel zeigt,
- Fig. 4: Querschnitte von Ausführungsbeispielen unterschiedlicher Materialien zeigt, die u.a. für das Ausführungsbeispiel gemäß den Fig. 1 bis 3 verwendet werden können,
- Fig. 5 und 6: Ergänzungen des in den Fig. 1 bis 3 gezeigten Ausführungsbeispiels zeigen,
- Fig. 7: eine Darstellung eines zu dem in den Fig. 1 bis 3 dargestellten Ausführungsbeispiel alternativen Ausführungsbeispiels,
- Fig. 8: eine perspektivische Darstellung eines Ausführungsbeispiels einer erfindungsgemässen Schiene,
- Fig. 9: Darstellungen von Stützelementen zur Verwendung in Ausführungsbeispielen von Schienen gemäß Fig. 1, 7 und 8 zeigt,
- Fig. 10: ein Detail eines Stützelements aus Fig. 9 zeigt,
- Fig. 11: eine Draufsicht auf eine nicht erfindungsgemässe Schiene zeigt,
- Fig. 12: einen Schnitt entlang der Linie XII - XII aus Fig. 11 zeigt,
- Fig. 13 und 14: Seitenansichten von Stützkörpern für aus dem Ausführungsbeispiel gemäß Fig. 13 zeigen,
- Fig. 15: eine weitere Ausführungsform des Materials der Körperelemente der Ausführungsbeispiele im Querschnitt zeigt und
- Fig. 16 und 17: Ausführungsformen des Materials für die Körperelemente der Ausführungsbeispiele in perspektivischer Darstellung zeigen.

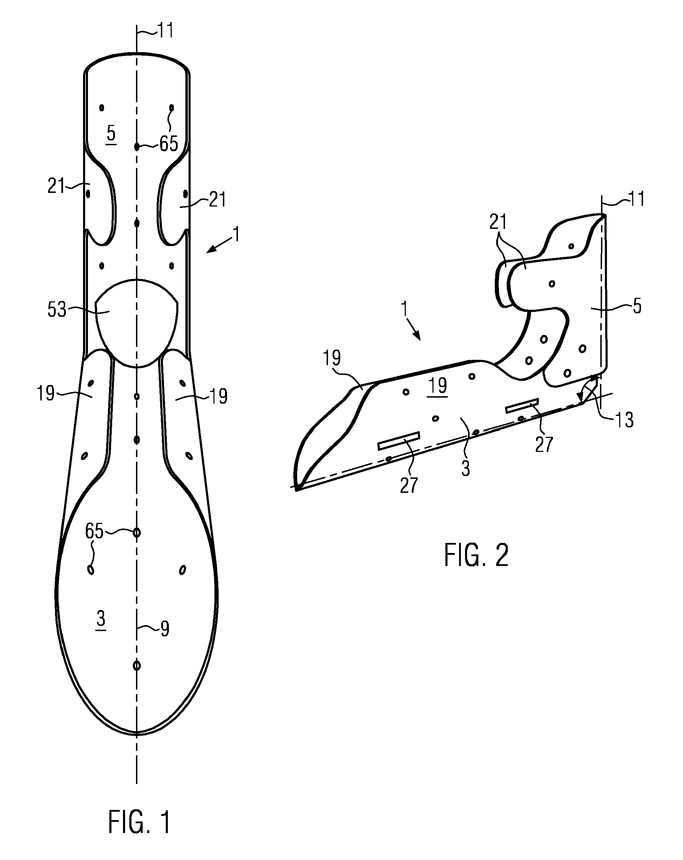

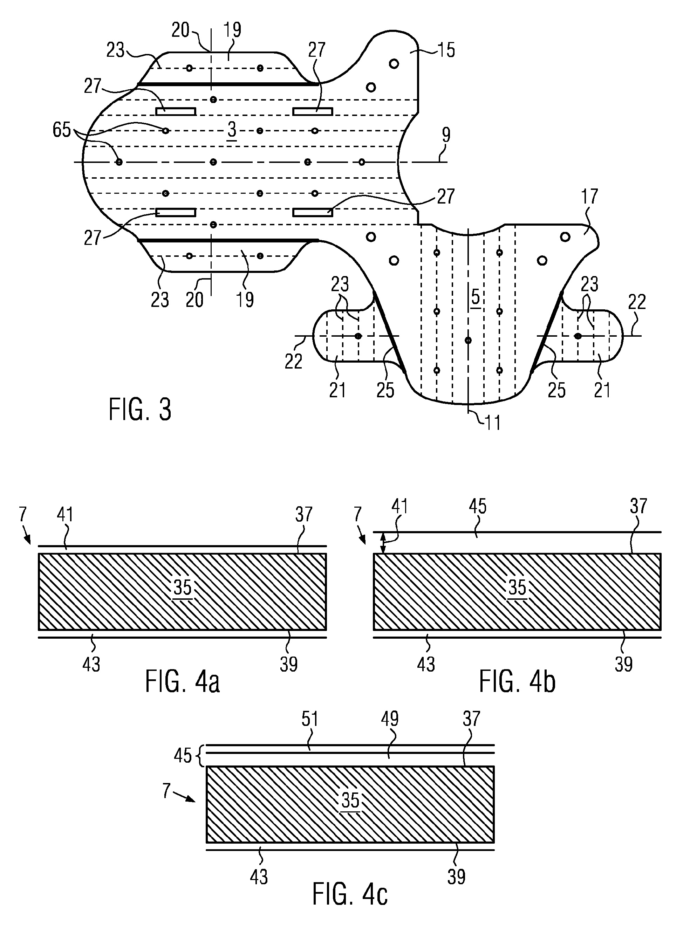

In den Figuren 1 bis 3 ist eine Schiene 1 dargestellt, wobei Figur 3 einen Zuschnitt zeigt, aus dem die in den Figuren 1 und 2 perspektivisch gezeigte Schiene 1 aufgefaltet werden kann.

Wie den Figuren zu entnehmen ist, weist die Schiene 1 ein erstes Körperelement 3 und ein zweites Körperelement 5 auf, die aus einem flächenförmigen, flexiblen Material 7 gebildet sind. Dabei ist im Zusammenhang mit der vorliegenden Erfindung unter dem Begriff eines "flächenförmigen" Materials oder Körpers ein solcher zu verstehen, dessen größte Abmessung in einer Ebene, in der der Körper verläuft, die Abmessung in der Richtung senkrecht zu dieser Ebene um wenigstens eine Größenordnung, vorzugsweise jedoch um mehr als eine Größenordnung übersteigt. Somit fallen unter diesen Begriff insbesondere Pappen, oder ähnliche aus Kunststoff gebildete, flächige Materialien

Wie des Weiteren aus den Figuren 1 bis 3 zu erkennen ist, sind sowohl das erste als auch das zweite Körperelement 3, 5 in einer Ebene, die sich senkrecht zu einer Längsachse 9, 11 des jeweiligen Körperelements 3, 5 erstreckt, U-förmig gebogen. Dabei verlaufen die so definierten Längsachsen 9, 11 in der Mitte des U-förmigen Querschnitts durch die Körperelemente 3, 5. Außerdem sind die Körperelemente 3, 5 derart miteinander verbunden, dass die Längsachsen 9, 11 im aufgefalteten Zustand einen Schienenwinkel 13 miteinander einschließen, wobei der Schienenwinkel 13 zwischen 80° und 100° und im hier dargestellten bevorzugten Ausführungsbeispiel bei etwa 90° liegt.

Im vorliegenden, in den Figuren 1 bis 3 dargestellten Ausführungsbeispiel erfolgt die Verbindung der Körperelemente 3, 5 in der Weise, dass beim Aufhalten des in Figur 3 dargestellten Zuschnitts die beiden freien Enden 15, 17 miteinander verbunden werden, so dass sich allein dadurch schon bei beiden Körperelementen 3, 5 der U-förmige Querschnitt ergibt. Es ist aber genauso denkbar, dass die Körperelemente 3, 5 aus getrennten Elementen statt aus einem einstückigen Zuschnitt gebildet sind, so dass die Körperelemente 3, 5 dann an zwei voneinander beabstandeten Stellen miteinander verbunden werden müssen. Hierbei ist es auch möglich, dass die Körperelemente 3, 5 schwenkbar miteinander verbunden sind, sodass der Schienenwinkel 13 eingestellt werden kann.

Wie weiter den Figuren 1 bis 3 zu entnehmen ist, weist sowohl das erste Körperelement 3 als auch das zweite Körperelement 5 auf beiden Seiten der Längsachse 9, 11 Laschen 19, 21 auf, die sich auf beiden Seiten der Längsachse 9, 11 davon weg erstrecken. In dem hier dargestellten, bevorzugten Ausführungsbeispiel erstrecken sich sowohl die Laschen 19 am ersten Körperelement 3 als auch die Laschen 21 am zweiten Körperelement 5 entlang einer jeweiligen Erstreckungsrichtung 20, 22 senkrecht von der jeweiligen Längsachse 9, 11 weg und weisen darüber hinaus sich parallel zur Längsachse 9, 11 erstreckende Falzlinien 23 auf, so dass die Laschen selbst in einfacher Weise parallel zu den Längsachsen 9, 11 gebogen werden können.

Des Weiteren ist das zweite Körperelement 5 derart bemessen, dass sich dessen Außenkanten 25 geneigt zu der Längsachse 11 des zweiten Körperelements 5 erstrecken.

Außerdem sind bei den in den Figuren 1 bis 3 dargestellten Ausführungsbeispiel in dem ersten Körperelement 3 beabstandet von der Längsachse 9 Aussparungen 27 vorgesehen, die eine längliche Form haben und derart in ersten Körperelement 3 angeordnet sind, dass die Richtung der größten Erstreckung der Aussparungen 27 parallel zur Längsachse 9 des ersten Körperelements 3 verläuft. Durch diese Aussparungen 27 können bei angelegter Schiene 1 nicht dargestellte Gurte geführt werden, mit denen die Schiene 1 beispielsweise an einem Arm oder Beinabschnitt eines Patienten befestigt wird.

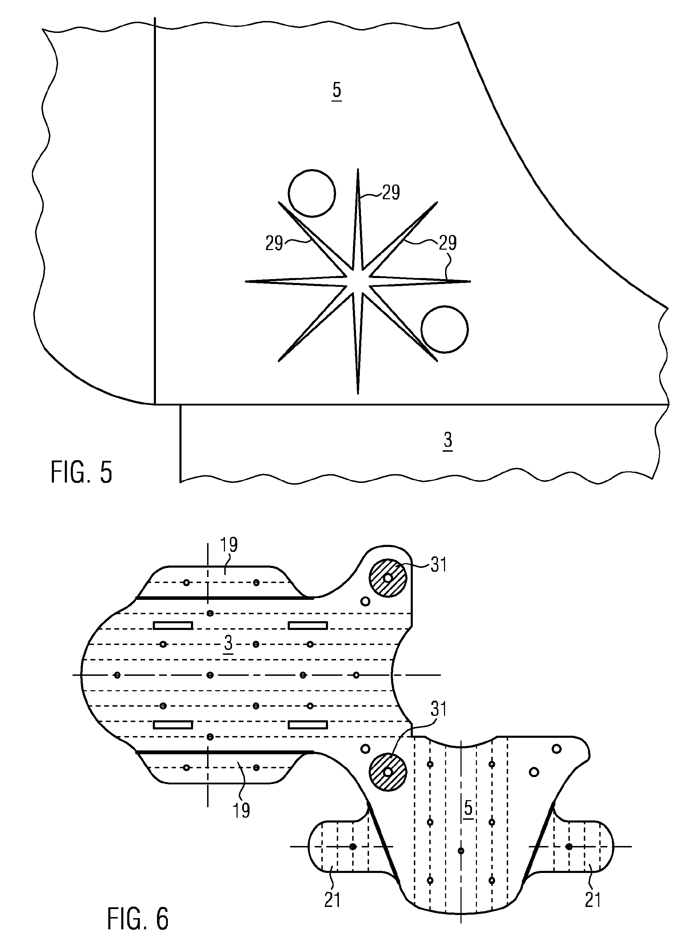

Daneben können abweichend von den in den Figuren 1 bis 3 dargestellten Ausführungsbeispiel im Bereich der Verbindung der Körperelemente 3, 5 Einschnitte 29 vorgesehen sein, die sternförmig verlaufen, so dass das Material der Körperelemente 3, 5 hier leicht nach außen gebogen werden kann, und in die Gefahr von Druckstellen insbesondere auf Gelenke eines gestützten Körperabschnitts eines Patienten vermieden werden (siehe Figur 5).

Alternativ zu den Einschnitten 29 ist es auch möglich, dass im Bereich der Verbindung der Körperelemente 3, 5 Schaumstoffelemente 31 auf der nach innen weisenden Seite der Schiene 1 angebracht sind, die dann ebenfalls im Bereich eines Gelenks zur Anlage mit dem Körperabschnitt kommen und so ebenfalls Druckstellen vermeiden. Diese Alternative ist in Figur 6 dargestellt.

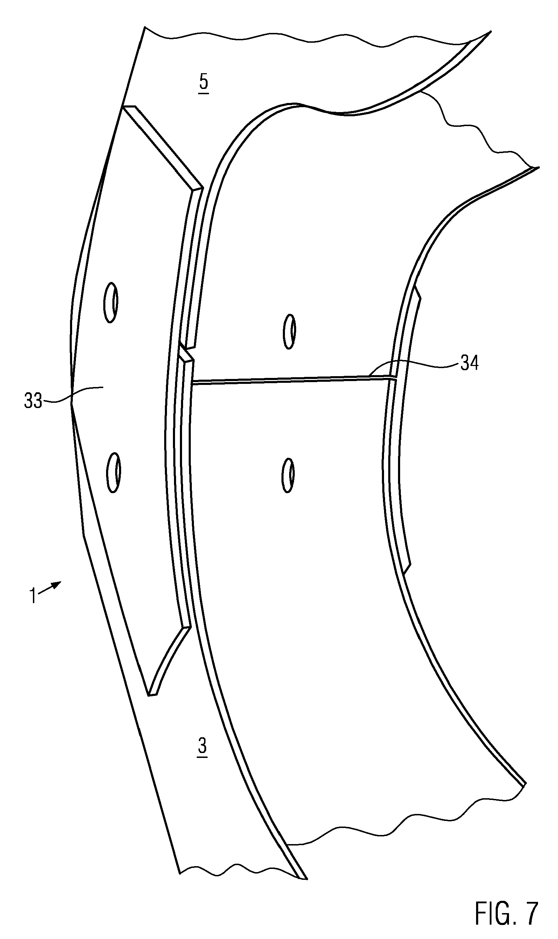

In Figur 7 ist ein Aufbau einer Schiene 1' dargestellt, bei der die Körperelemente 3, 5 getrennt voneinander sind und nicht in einem einstückig ausgebildeten Zuschnitt geformt sind, wie dies bei dem in den Figuren 1 bis 3 dargestellten Ausführungsbeispiel der Fall war. Auch dieses Ausführungsbeispiel weist U-förmig um eine Längsachse 9, 11 gebogenen Körperelemente 3, 5 auf, an deren von der Längsachse 9, 11 abgewandten Kanten sich senkrecht dazu erstreckende Laschen 19, 21 ausgebildet sind. Darüber hinaus sind die Körperelemente 3, 5 in der Weise miteinander verbunden, dass zwei Verbindungselemente 33 vorgesehen sind, die auf der nach außen weisenden Oberfläche der Köperelemente 3, 5 befestigt sind, so dass zueinander weisende Kanten der Körperelemente 3, 5 in einer Ebene einander gegenüberliegen, also ein Stumpfstoß 34 gebildet wird. Dies hat insbesondere zur Folge, dass die nach innen weisenden Oberflächen der Körperelemente 3, 5 ebenfalls in einer gemeinsamen Ebene verlaufen und es keine Stufen gibt, die sonst zu Druckstellen beim Patienten führen könnten.

In Figur 4 sind Ausführungsbeispiele von Materialien im Querschnitt dargestellt, die insbesondere für die in den Figuren 1 bis 3 sowie 7 dargestellten Ausführungsbeispiele von Schienen 1, 1' verwendet werden können. Diesen Materialien können aber auch für andere aus flächenförmigen Material hergestellte Schienen verwendet werden, so dass die Anwendung dieser im Folgenden beschriebenen Materialien nicht auf die ansonsten hierin beschriebenen Schienen beschränkt ist.

Diese Materialien 7 weisen zunächst alle einen flächenförmigen, flexiblen Grundkörper 35 auf, der eine im aufgefalteten Zustand der Schiene zum Körperabschnitt weisende Anlageoberfläche 37 und eine von der Anlageoberfläche 37 wegweisende Außenoberfläche 39 aufweist. Gemäß dem in Figur 4a) gezeigten Ausführungsbeispiel eines Materials 7 ist auf der Anlageoberfläche 37 eine antimikrobiell wirkende Beschichtung 41 aufgebracht, die als Acetat-Träger ausgebildet sein kann, der beispielsweise auf die Anlageoberfläche 37 aufkaschiert ist.

Alternativ dazu kann die antimikrobiell wirkende Beschichtung 41 als Wirksubstanz Dialkylcarbamoylchlorid (DACC) und/oder Polyhexamethylenbiguanid (PHMB) und/oder Silber aufweisen. Dabei kann die Wirksubstanz in ein Vlies, Gewebe, Gestrick oder Gewirk eingebracht sein, das dann auf der Anlageoberfläche 37 als Beschichtung 41 aufkaschiert wird. Hierbei ist es vorteilhaft, wenn das Vlies, Gewebe, Gestrick oder Gewirk bereits mit der Wirksubstanz ausgerüstet worden ist.

Auf der von der Anlageoberfläche 37 abgewandten Außenoberfläche 39 ist bei allen in Figur 4 dargestellten Ausführungsbeispielen von einem Material zur Verwendung bei einer Schiene 1 eine Schicht 43 aufgebracht, die mit einem Gewebe, insbesondere einem Verbandmaterial, eine gegenüber dem Grundköper 35 erhöhter Haftreibung hat. Dabei kann diese Schicht 43 als eine Lackierung aufgebracht sein. Es ist aber auch denkbar, dass die die Haftreibung erhöhende Schicht 43 aufkaschiert ist.

In einem besonders bevorzugten Ausführungsbeispiel weist die Schicht 43 ein gitterförmig verlaufendes Material auf, das vorzugsweise aus einem polymeren Werkstoff gebildet ist, was mit dem Vorteil verbunden ist, dass das Gitter einen guten Eingriff mit einem um die Schiene 1 gewickelten Verbandmaterial schafft, das ein Verrutschen des Verbandsmaterials verhindert.

Schließlich ist es auch möglich, dass die Schicht 43 als Hakenelement einer Haken-Schlaufen-Verbindung ausgebildet ist. Auch eine solche, dann nur teilweise auf der Außenoberfläche vorgesehene Schicht 43 führt zu einem guten Eingriff zwischen Schiene 1 und Verbandmaterial.

In den Figuren 4b und c ist dargestellt, dass auf der zum Körperabschnitt weisenden Anlageoberfläche 37 des Grundkörpers 35 eine Absorptionsschicht 45 vorgesehen ist, die zur Aufnahme von Wundexsudat dient. Gemäß dem in Figur 4 b) dargestellten Ausführungsbeispiel ist die Absorptionsschicht 45 als Schaum ausgeführt, wobei es bevorzugt ist, einen offenzelligen Polyurethanschaum zu verwenden, dessen Dichte zwischen 30 und 110 kg/m³ liegt und/oder dessen Dicke 47 zwischen 2,5 und 15 mm liegt. Bei einer solchen Wahl wird eine Kapazität der Absorptionsschicht 45 erreicht, die ausreichend ist, normalerweise austretende Wundexsudatmengen aufzunehmen.

Bei dem in Figur 4 c) dargestellten Ausführungsbeispiel ist die Absorptionsschicht 45 derart als Fliesverbundschicht ausgebildet, dass eine Viskoseschicht 49 vorgesehen ist, die unmittelbar auf der Anlageoberfläche 37 aufgebracht ist, wobei diese wiederum von einer Polyesterschicht 51 überzogen ist. Ein derartiger Aufbau stellt sicher, dass einmal von der Absorptionsschicht 45 aufgenommenes und in der Viskoseschicht 49 gespeichertes Exsudat nicht wieder abgegeben wird, was effektiv durch die Polyesterschicht 51 vermieden wird.

Die zuvor beschriebenen Ausführungsbeispiele von 1, 1' können so verwendet werden, dass beispielsweise der Arm oder das Bein eines Patienten von der Schiene 1, 1' in der Weise aufgenommen wird, dass beispielsweise der Unterarm im ersten Körperelement 3 zu liegen kommt, während der Oberarm vom zweiten Körperelement 5 aufgenommen wird. Der Ellenbogen kann sich dann durch die zwischen den Körperelementen 3, 5 vorgesehene Öffnung 53 erstrecken, und der Arm wird durch die Laschen 19, 21 sicher fixiert, die aufgrund der Falzlinien darum gebogen werden können. Darüber hinaus können durch die Aussparungen 27 Gurte geführt werden, die die Schienen 1, 1' weiter festlegen.

Alternativ zu den Gurten ist es auch möglich Verbandmaterial um die Schiene 1 zu wickeln, wobei die auf der Außenoberfläche 39 der Körperelemente 3, 5 vorgesehene Schicht 43 ein Verrutschen des Verbandmaterials zuverlässig verhindert.

Wenn auf der Anlageoberfläche 37 eine antimikrobiell wirkende Beschichtung 41 vorgesehen ist, wird bei längerem Tragen der Schiene 1, 1' zuverlässig vermieden, dass sich am Körperabschnitt des Patienten, der von der Schiene 1, 1' gestützt wird, Entzündungen entwickeln können.

Alternativ dazu führt eine Absorptionsschicht 45 dazu, dass möglicherweise von Wunden abgegebenes Wundexsudat von dieser Schicht 45 aufgenommen und gehalten wird, so dass es nicht zu einem Aufweichen des Grundkörpers 35 des Materials 7 der Schiene 1, 1' kommen kann. Außerdem wird vermieden, dass sich Mazerationen beim Patienten bilden können.

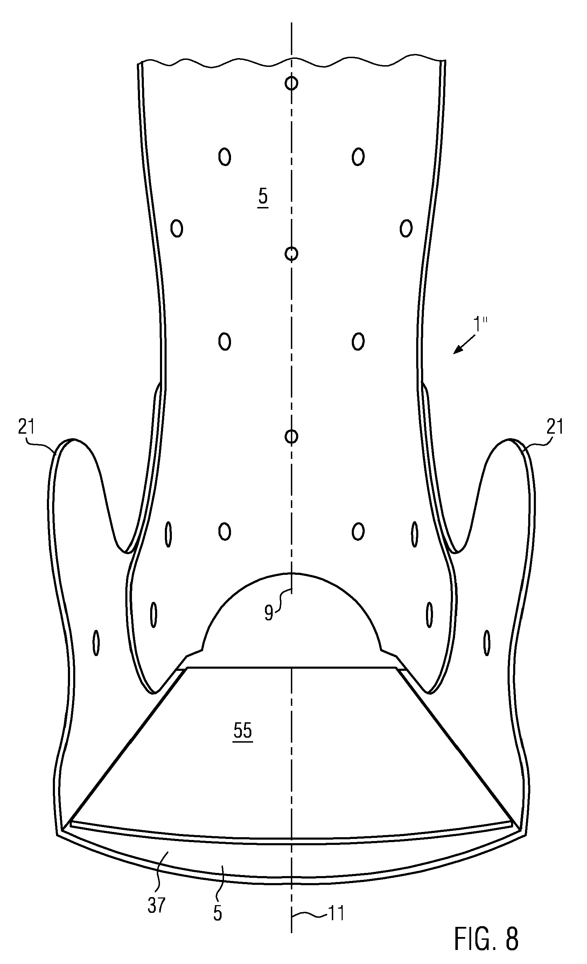

In Figur 8 ist eine erfindungsgemässe Schiene gezeigt, wobei hier das erste Körperelement 3 und das zweite Köperelement 5 als getrennte Elemente ausgebildet sind, die nicht in einem gemeinsamen einstückig ausgebildeten Zuschnitt enthalten sind. Auch hier weist das zweite Körperelement 5 Laschen 21 auf, die im vorliegenden Ausführungsbeispiel dazu genutzt werden können, den Fuß eines Patienten zu fixieren.

Wie weiter in Figur 8 zu sehen ist, ist auf der Anlageoberfläche 37 des zweiten Körperelements 5 ein Zusatzelement 55 vorgesehen, dass sich über einen Großteil der Anlageoberfläche 37 erstreckt und dazu dient, die Stabilität des zweiten Köperelements 5 zu erhöhen. Dies ist insbesondere dann sinnvoll, wenn die Schiene 1 zum Stützen eines Fußes bzw. Schienbeins eingesetzt wird, wobei der Fuß in dem zweiten Körperelement 5 aufgenommen ist und der Benutzer auf den Boden auftritt, so dass das zweite Körperelement 5 diese Belastungen aufnehmen muss.

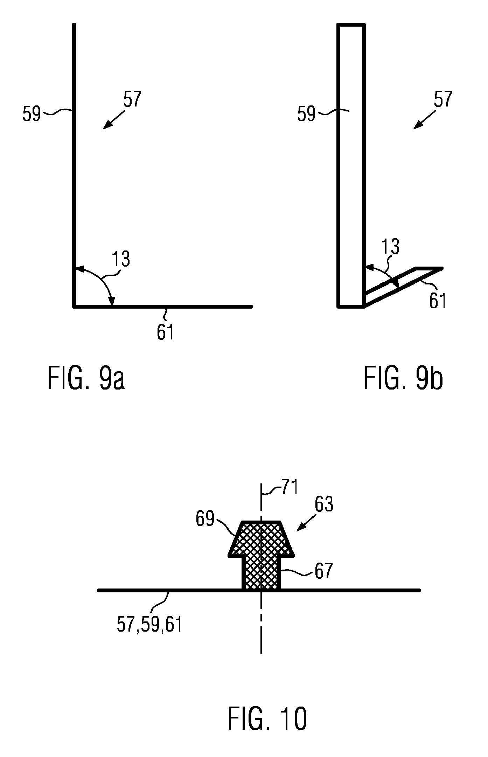

In Figur 9 ist ein Stützkörper 57 in Seitenansicht (Teil a)) und perspektivischer Darstellung (Teil b)), der in einer Schiene 1, 1' verwendet werden kann, wie sie im Zusammenhang mit den Figuren 1, 7 und 8 beschrieben worden ist. Der Stützkörper 57 ist stabförmig ausgebildet und weist einen ersten Stababschnitt 59 und einen zweiten Stababschnitt 61 auf, wobei die Stababschnitte 59, 61 in einem Winkel zueinander angeordnet sind.

Unter dem Begriff "stabförmig" im Sinne der vorliegenden Erfindung fallen solche Körper, deren Abmessung in einer durch den Körper definierten Erstreckungsrichtung wenigstens um eine Größenordnung größer ist als die Abmessungen senkrecht zur Erstreckungsrichtung.

Der Stützkörper 57 kann an der Außenoberfläche 39 der Körperelemente 3, 5 der Schiene 1, 1' anliegen und dadurch den Schienenwinkel 13, unter dem die Längsachsen 9, 11 der Körperelemente 3, 5 verlaufen, vorgeben. Die ist dann von Bedeutung, wenn die Körperelemente 3, 5 schwenkbar miteinander verbunden sind, so dass der Schienenwinkel 13 frei einstellbar ist.

Das Material des Stützkörpers 57 kann ein thermoplastisches Material sein und insbesondere ein thermoplastischer Kunststoff, dessen Erweichungstemperatur im Bereich zwischen 50°C und 200°C, bevorzugt zwischen 60°C und 170°C und am meisten bevorzugt zwischen 70°C und 140°C liegt. Dann kann der Stützkörper 57 zunächst soweit erwärmt werden, dass er aufgrund des Überschreitens der Erweichungstemperatur verformbar ist, so dass dann der Schienenwinkel 13 eingestellt werden kann. Nach dem Abkühlen wird der Stützkörper 57 an die Körperelemente 3, 5, die ggf. schwenkbar miteinander verbunden sind, angelegt, so dass der dann ausgehärtete Stützkörper 57 den Schienenwinkel 13 festlegt.

Dies ermöglicht, dass ein Sanitäter oder Arzt in einfacher Weise den Schienenwinkel vorgeben kann, die Schiene dann aber trotzdem aufgrund des steifen Stützkörpers 57 eine hohe Stabilität aufweist.

Alternativ kann es sich bei dem Material des Stützkörpers 57 auch um einen Vliesstoff handeln, wobei hier ein Gemisch aus 40% Baumwolle, 30% Polypropylen und 30% Polyester bevorzugt ist (die Angaben beziehen sich auf die Gewichtsprozente). Ein Vliesstoff, der dabei eine Grammatur von mehr als 500g/m² hat, kann in besonders bevorzugter Weise verwendet werden und ergibt einen Stützkörper mit hervorragender Stabilität.

Wie schließlich in Figur 10 gezeigt ist, können an dem Stützkörper 57 auf der zur Außenoberfläche 39 der Schiene 1, 1' weisende Seite Befestigungseinrichtungen in Form von Befestigungselementen 63 angebracht sein. Die Befestigungselemente 63 können mit Löchern 65 in den Körperelementen 3, 5 der Schiene 1 eingreifen und sind derart ausgebildet, dass sie einen ersten Abschnitt 67 aufweisen, der mit dem Stützelement 57 bzw. einem Stababschnitt 59, 61 verbunden ist, und einen zweiten Abschnitt 69, der am vom Stützkörper 57 bzw. Stababschnitt 59, 61 entfernten Ende des ersten Abschnitts 67 angeordnet ist. Der erste Abschnitt 67 und der zweite Abschnitt 69 weisen derartige Abmessungen auf, dass die erste Abmessung des erste Abschnitts 67 senkrecht zu der Erstreckungsrichtung 71 des Befestigungselements kleiner ist als die zweite Abmessung des zweiten Abschnitts 69 senkrecht zur Erstreckungsrichtung 71. Somit wird eine im Wesentlichen pilzförmiges Befestigungselement 63 geschaffen, wobei der zweite Abschnitt 69 dann, wenn das Befestigungselement 63 durch ein Loch 65 in einem Körperelement 3,5 hindurchgestreckt wird, das Körperelement 3,5 hintergreifen kann, so dass der Stützkörper 57 festgelegt wird. Auf diese Weise kann das Stützelement 57 dann leicht an dem Körperelement befestigt werden. Dabei ist es bevorzugt, wenn das Befestigungselement 63 aus einem elastischen Kunststoff wie beispielsweise Silikon gebildet ist.

Alternativ zu den zuvor beschriebenen Befestigungselementen 63 ist es aber auch möglich, dass sowohl der Stützkörper 57 als auch die Körperelemente 3, 5 auf der Außenoberfläche 39 Elemente einer Haken-Schlaufen-Verbindung aufweisen, so dass das Stützelement 57 mittels eines "Klettverschlusses" an den Körperelementen 3, 5 befestigt werden kann.

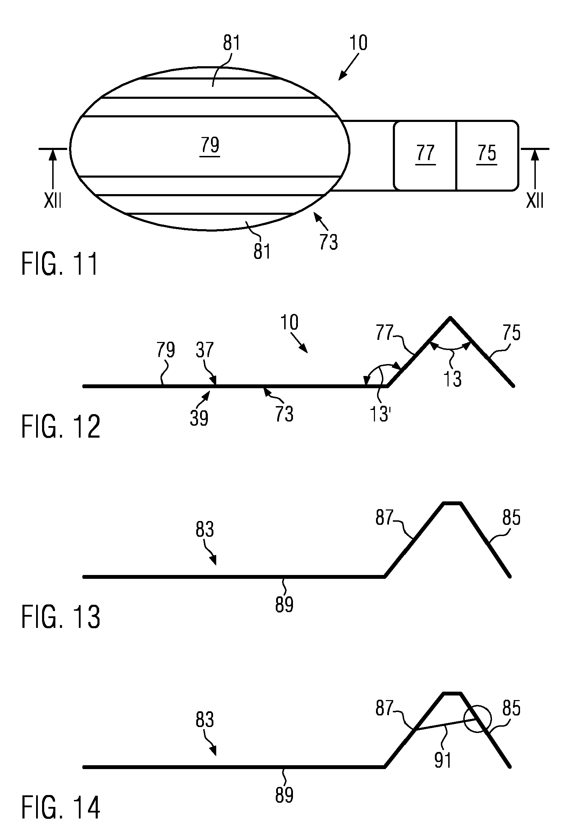

In den Fig. 11 bis 14 ist eine nicht erfindnugsgemässe Schiene 10 dargestellt, wobei die Schiene 10 ein flächenförmiges Körperelement 73 aufweist, das einen ersten Anlageabschnitt 75, einen zweiten Anlageabschnitt 77 sowie einen dritten Anlageabschnitt 79 aufweist, an den ein Körperabschnitt eines Patienten anliegen kann.

Das Material des Körperelements 73 kann dem entsprechen, das in Zusammenhang mit Fig. 4 beschrieben worden ist. Es ist also auch hier möglich, dass eine antimikrobielle Beschichtung 41, eine Schicht 43 mit erhöhter Haftreibung oder eine Absorptionsschicht 45 vorgesehen sind.

Das Körperelement 73 ist derart ausgestaltet, dass am dritten Anlageabschnitt 79 seitliche Laschen 81 mit parallel zur Erstreckungsrichtung des Körperelements 73 verlaufenden Falzlinien 82 vorgesehen sind, die aufgrund der Flexibilität des Materials des Körperelements 73 hochgebogen werden können. Außerdem ist die Schiene 10 derart ausgestaltet, dass zwischen den Anlageabschnitten 75, 77, 79 jeweils ein Schienenwinkel 13, 13' ausgebildet ist, die Abschnitte also nicht parallel zueinander oder in einer Ebene verlaufen.

In den Fig. 13 ist ein erstes Beispiel des Stützkörpers 83 der Schiene 10 dargestellt, der aus einem stabförmigen Material gebildet ist, das starr ist und nur eine geringe Elastizität aufweist. Der Stützkörper umfasst erste, zweite und dritte Stababschnitte 85, 87, 89 und ist derart geformt, dass die Stababschnitte 85, 87, 89 jeweils unter einem Winkel zueinander angeordnet sind, wobei der Winkel zwischen dem ersten Stababschnitt 85 und dem zweiten Stababschnitt 87 dem Schienenwinkel 13 zwischen dem ersten Anlageabschnitt 75 und dem zweiten Anlageabschnitt 77 entspricht. In gleicher Weise entspricht der Winkel zwischen dem zweiten Stababschnitt 87 und dem dritten Stababschnitt 89 dem Winkel 13' zwischen dem zweiten Anlageabschnitt 77 und dem dritten Anlageabschnitt 79.

In montiertem Zustand liegt der Stützkörper 83 auf der der Anlageoberfläche 37 abgewandten Außenoberfläche 39 des Körperelements 73 an, so dass der Stützkörper 83 den Schienenwinkel 13 sowie den Winkel 13' zwischen dem zweiten und dritten Anlageabschnitt 77, 79 vorgibt.

Auch in diesem Fall kann der Stützkörper 83 wieder aus einem thermoplastischen Kunststoff mit einer Erweichungstemperatur zwischen 50°C und 200°C, bevorzugt zwischen 60°C und 170°C und besonders bevorzugt zwischen 70°C und 140°C gebildet sein, so dass die Winkel 13, 13' in einfacher Weise durch Erwärmen des Stützkörpers 83 eingestellt werden können. Somit hat auch bei der hier dargestellten Schiene 10 der Arzt oder Sanitäter eine große Flexibilität beim Einstellen der Schiene. Auch in diesem Fall kann der Stützkörper 83 wieder aus einem Vliesstoffgemisch aus Baumwolle, Polypropylen und Polyestern gebildet sein, wobei vorzugsweise 40% Baumwolle, 30% Polypropylen und 30% Polyester gemischt sind (Angabe der Gewichtsprozente).

Bei dem in Fig. 14 dargestellten zweiten Ausführungsbeispiel eines Stützkörpers 83 ist zusätzlich ein Verbindungselement 91 vorgesehen, das mit dem ersten und zweiten Stababschnitt 85, 87 jeweils beabstandet von dem Punkt verbunden ist, wo die Stababschnitte 85, 87 miteinander verbunden sind. Auf diese Weise wird durch das Verbindungselement 91 der Winkel zwischen den Stababschnitten 85, 87 und damit der Schienenwinkel in zuverlässiger Weise festgelegt.

Schließlich können auch bei diesem Ausführungsbeispiel der Stützkörper 83 und das Körperelement 73 entweder über die in Fig. 10 gezeigten Befestigungselemente 63 am Stützkörper 83 und entsprechende Löcher im Körperelement 73 verbunden werden. Alternativ kann auch eine Haken-Schlaufen-Verbindung zwischen beiden vorgesehen sein. Dies ermöglicht jeweils eine lösbare Verbindung zwischen Körperelement 73 einerseits und Stützkörper 83 andererseits, so dass das Körperelement 73 in Form eines ebenen Zuschnitts platzsparend transportiert und gelagert werden kann.

Die Schiene 10 kann insbesondere an den Unterarm eines Patienten in der Weise angelegt werden, dass der erste und zweite Anlageabschnitt 75, 77 die Handfläche stützen, während der Unterarm selbst auf dem dritten Anlageabschnitt 89 aufliegt, wobei der Stützkörper 83 die Winkel zwischen den Anlageabschnitten 75, 77, 83 vorgibt. Wenn eine antimikrobielle Beschichtung 41 oder eine Absorptionsschicht 45 vorgesehen sind, werden Entzündung oder ein Aufweichen des Materials des Körperelements sowie Mazerationen am Patienten vermieden, wenn die Schiene 10 mittels eines Verbandmaterials fixiert ist.

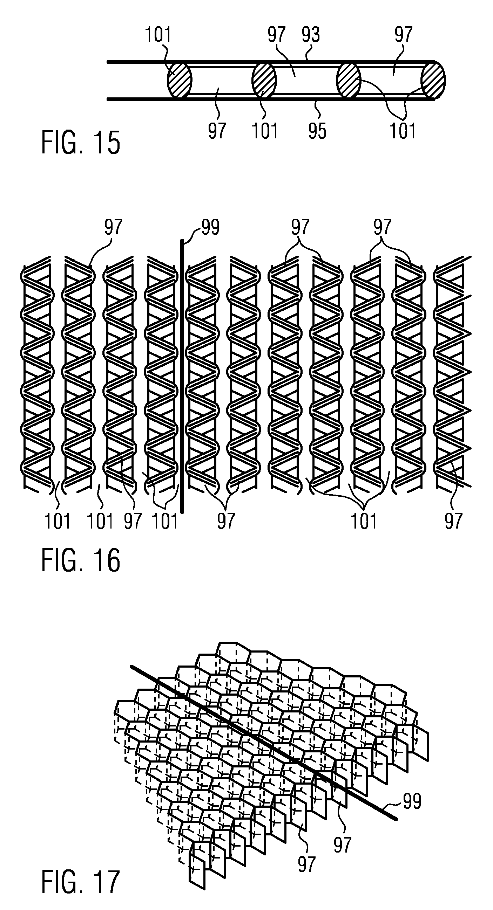

In Fig. 15 ist eine weitere Ausführungsform eines Materials für den Grundkörper 35 (s. Figur 4) für die Körperelemente 3, 5, 73 dargestellt, wobei das Material flächenförmig und biegbar ist und eine erste und eine zweite Schicht 93, 95 aufweist. Die Schichten 93, 95 erstrecken sich in der Ebene des Körperelements 3, 5, 73 und bilden die Anlageoberfläche 37 bzw. die Außenoberfläche 39. Die Schichten 93, 95 können aus Pappe oder aus Polypropylen gebildet sein. Es sind aber auch andere Materialien wie beispielsweise Folien denkbar. Auch ist es möglich, dass die erste und zweite Schicht 93, 95 durch ein Vlies gebildet werden.

Zwischen der ersten und zweiten Schicht 93, 95 sind Streifen angeordnet, die in Fig. 16 perspektivisch dargestellt sind. Zum einen verlaufen zwischen den Schichten 93, 95 erste Streifen 97 mäanderförmig entlang einer Verlaufsrichtung 99. Durch die ersten Streifen wird eine Honigwaben-ähnliche Struktur gebildet. Neben den mäanderförmig verlaufenden ersten Streifen 97 sind auch noch gradlinig, ebenfalls entlang der Verlaufsrichtung 99 verlaufenden zweiten Streifen 101 vorgesehen, die das Material weiter stabilisieren.

Ein derartiger Aufbau des Materials führt dazu, dass die Körperelemente 3, 5, 73 um Linien, die parallel zur Verlaufsrichtung 99 verlaufen leicht biegbar sind, während die Körperelemente jedoch in Richtung der Verlaufsrichtung 99 eine sehr hohe Biegesteifigkeit aufweisen.

In Fig. 17 ist ein weiteres Ausführungsbeispiel für das Material der Körperelemente 3, 5, 73 dargestellt, wobei hier im Unterschied zu der dem in Fig. 16 dargestellten keine gradlinig verlaufenden Streifen 101 vorgesehen sind, sondern lediglich mäanderförmig verlaufende Streifen 97, die sich senkrecht zur ersten und zweiten Schicht 93, 95 erstrecken, so dass sich hier im Zwischenraum zwischen den Schichten 93, 95 ebenfalls eine Honigwabenstruktur ergibt. Auch dieses Material weist in Richtung der Verlaufsrichtung 99 der ersten Streifen 97 eine hohe Biegesteifigkeit auf, während in der Richtung senkrecht dazu das Material leicht gebogen werden kann.

Wie sich aus dem Vorstehenden ergibt, werden durch die erfindungsgemäßen Schienen 1, 1' 10 die eingangs genannten Nachteile des Standes der Technik beseitigt.

## Patentansprüche

1. Schiene zum Stützen eines Fußes und Schienbeins eines Patienten mit einem ersten Körperelement (3) und einem zweiten Körperelement (5), wobei das zweite Körperelement (5) zur Aufnahme eines Fußes des Patienten ausgebildet ist,
wobei die Körperelemente (3, 5) aus einem flächenförmigen flexiblen Material gebildet sind,
wobei das erste und das zweite Körperelement (3, 5) in einer Ebene senkrecht zu einer Längsachse (9, 11) des Körperelements (3, 5) einen im wesentlichen U-förmigen Querschnitt aufweisen, wobei die Längsachsen (9, 11) in der Mitte des U-förmigen Querschnitts durch die Körperelemente (3, 5) verlaufen,
wobei die Körperelemente (3, 5) derart miteinander verbunden sind, dass die Längsachse (9) des ersten Körperelements (3) und die Längsachse (11) des zweiten Körperelements (5) einen Schienenwinkel (13) miteinander einschließen, und
wobei die Körperelemente (3, 5) eine zum Inneren des U-förmigen Querschnitts weisende Anlageoberfläche (37) und eine der Anlageoberfläche (37) gegenüber liegende Außenoberfläche (39) aufweisen,
**dadurch gekennzeichnet,**
**dass** das Körperelement (5), das zur Aufnahme eines Fußes des Patienten ausgebildet ist, ein sich parallel dazu erstreckendes flächenförmiges Zusatzelement (55)
zur Erhöhung der Stabilität des zweiten Körperelementes aufweist, wobei das Zusatzelement (55) auf der Anlageoberfläche (37) des Fußes anliegt.

2. Schiene nach Anspruch 1, wobei das Material eine erste Schicht (93) und eine zweite Schicht (95) aufweist, die beabstandet und parallel zueinander verlaufen,
wobei zwischen der ersten und zweiten Schicht (93, 95) Streifen (97) vorgesehen sind und
wobei die Streifen (97) mäanderförmig entlang einer Verlaufsrichtung (99) verlaufen.

3. Schiene nach Anspruch 2, wobei die Streifen (97) flächenförmig ausgebildet sind und wobei sich die Streifen (97) senkrecht zu der ersten und der zweiten Schicht (93, 95) erstrecken.

4. Schiene nach Anspruch 2 oder 3, wobei die erste und/oder zweite Schicht (93, 95) aus Pappe oder Polypropylen gebildet sind.

5. Schiene nach Anspruch 2 oder 3, wobei die erste und/oder zweite Schicht (93, 95) ein Vlies aufweisen.

6. Schiene nach einem der Ansprüche 2 bis 5, wobei zweite Streifen (101) vorgesehen sind, die sich geradlinig entlang der Verlaufsrichtung (99) zwischen den mäanderförmigen Streifen (97) abgeordnet sind.

## Claims

1. A splint for supporting a foot and a shin of a patient by means of a first body element (3) and a second body element (5), wherein the second body element (5) is designed for receiving a patient's foot,
wherein the body elements (3, 5) are formed from a flat, flexible material,
wherein the first and second body elements (3, 5) have a substantially U-shaped cross-section in a plane perpendicular to a longitudinal axis (9, 11) of the body element (3, 5), wherein the longitudinal axes (9, 11) run in the middle of the U-shaped cross-section through the body elements (3, 5),
wherein the body elements (3, 5) are connected to each other such that the longitudinal axis (9) of the first body element (3) and the longitudinal axis (11) of the second body element (5) enclose a splint angle (13), and
wherein the body elements (3, 5) have a bearing surface (37) facing the inside of the U-shaped cross-section and an outer surface (39) opposite the bearing surface (37),
**characterized in**
**that** the body element (5) which is designed for receiving a patient's foot has a flat additional element (55) extending parallel thereto in order to increase the stability of the second body element, wherein the additional element (55) abuts on the bearing surface (37) of the foot.

2. The splint according to claim 1, wherein the material has a first layer (93) and a second layer (95) that proceed at a distance and parallel to each other,
wherein strips (97) are provided between the first and second layers (93, 95), and
wherein the strips (97) meander along a path (99).

3. The splint according to claim 2, wherein the strips (97) are designed flat and wherein the strips (97) extend perpendicular to the first and second layers (93, 95).

4. The splint according to claim 2 or 3, wherein the first and/or second layer/s (93, 95) are made of cardboard or polypropylene.

5. The splint according to claim 2 or 3, wherein the first and/or second layers (93, 95) have a non-woven fabric.

6. The splint according to one of claims 2 to 5, wherein second strips (101) are provided that are arranged in a straight line along the path (99) between the meandering strips (97).

## Revendications

1. Rail pour le support d'un pied et du tibia d'un patient avec un premier élément de corps (3) et un deuxième élément de corps (5), le deuxième élément de corps (5) étant conçu pour le logement d'un pied du patient,
les éléments de corps (3, 5) étant constitués d'un matériau flexible formant une surface,
le premier et le deuxième élément de corps (3, 5) présentant, dans un plan perpendiculaire à un axe longitudinal (9, 11) des éléments de corps (3, 5), une section transversale globalement en forme de U, les axes longitudinaux (9, 11) s'étendant au centre de la section transversale en forme de U à travers les éléments de corps (3, 5),
les éléments de corps (3, 5) étant reliés entre eux de façon à ce que l'axe longitudinal (9) du premier élément de corps (3) et l'axe longitudinal (11) du deuxième élément de corps (5) forment entre eux un angle de rail (13) et
les éléments de corps (3, 5) présentant une surface d'appui (37) orientée vers l'intérieur de la section transversale en forme de U et une surface extérieure (39) se trouvant en face de la surface d'appui (37),
**caractérisé en ce que**
l'élément de corps (5), qui est conçu pour le logement d'un pied d'un patient, comprend un élément supplémentaire (55) plat s'étendant parallèlement à celui-ci pour l'augmentation de la stabilité du deuxième élément de corps, l'élément supplémentaire (55) s'appuyant contre la surface d'appui (37) du pied.

2. Rail selon la revendication 1, le matériau comprenant une première couche (93) et une deuxième couche (95) qui s'étendant parallèlement et à une certaine distance l'une de l'autre,
des bandes (97) étant prévues entre la première et la deuxième couche (93, 95) et
les bandes (97) s'étendant en formant des méandres le long d'une direction d'extension (99).

3. Rail selon la revendication 2, les bandes (97) étant plates et les bandes (97) s'étendant perpendiculairement à la première et à la deuxième couche (93, 95).

4. Rail selon la revendication 2 ou 3, la première et/ou la deuxième couche (93, 95) étant constituées de papier ou de polypropylène.

5. Rail selon la revendication 2 ou 3, la première et/ou la deuxième couche (93, 95) comprenant un tissu non-tissé.

6. Rail selon l'une des revendications 2 à 5, des deuxièmes bandes (101) étant prévues, qui s'étendent linéairement le long de la direction d'extension (99) entre les bandes (97) formant des méandres.
